# EUROPÄISCHE PATENTSCHRIFT

(11) **EP 1 352 074 B1**
(45) Veröffentlichungstag und Bekanntmachung des Hinweises auf die Patenterteilung: **20.07.2011**
(21) Anmeldenummer: 02719687.2
(22) Anmeldetag: 10.01.2002
(51) Int. Cl.: C12N 15/68

(54) **VERFAHREN ZUR HERSTELLUNG VON HOMOGENEN NUKLEINSÄURE MULTIMEREN FÜR PHARMAZEUTISCHE ZUSAMMENSETZUNGEN**
METHOD FOR PRODUCING HOMOGENEOUS NUCLEIC ACID MULTIMERS FOR USE IN PHARMACEUTICAL COMPOSITIONS
PROCEDE PERMETTANT LA PRODUCTION DE MULTIMERES D'ACIDE NUCLEIQUE HOMOGENES DESTINES A DES COMPOSITIONS PHARMACEUTIQUES

(30) Priorität: 16.01.2001 DE 10101761
(43) Veröffentlichungstag der Anmeldung: 15.10.2003
(73) Patentinhaber: Plasmid Factory GmbH & Co. KG, 33607 Bielefeld (DE)
(72) Erfinder: VOSS, Carsten, 32791 Lage (DE); SCHMIDT, Torsten, 32791 Lage (DE); SCHLEEF, Martin, 33739 Bielefeld (DE); FRIEHS, Karl, 31319 Sehnde (DE); FLASCHEL, Erwin, 33619 Bielefeld (DE)
(74) Vertreter: Grund, Martin
(86) Internationale Anmeldenummer: PCT/EP2002/000194
(87) Internationale Veröffentlichungsnummer: WO 2002/062987

(56) Entgegenhaltungen:
- BEDBROOK J R ET AL: "RECOMBINATION BETWEEN BACTERIAL PLASMIDS LEADING TO THE FORMATION OF PLASMID MULTIMERS" CELL, Bd. 9, Nr. (4 PART 2, 1976, Seiten 707-716, XP001088105 ISSN: 0092-8674
- ARTHUR A ET AL: "DISSECTION OF THE TRANSPOSITION PROCESS A TRANSPOSON ENCODED SITE SPECIFIC RECOMBINATION SYSTEM" MOLECULAR & GENERAL GENETICS, Bd. 175, Nr. 3, 1979, Seiten 267-274, XP002205188 ISSN: 0026-8925
- SCHLEEF M ET AL: "Plasmid DNA for pharmaceutical applications." DEVELOPMENTS IN BIOLOGICALS. SWITZERLAND 2000, Bd. 104, 2000, Seiten 25-31, XP001088009 ISSN: 1424-6074

## Beschreibung

### Anwendungsgebiet

Gegenstand der vorliegenden Erfindung ist ein Verfahren zur Herstellung von homogenen Nukleinsäure-Multimeren für pharmazeutische Zusammensetzungen. Die homogenen Nukleinsäure-Multimere können in großen Mengen ohne Verwendung toxischer Substanzen hergestellt werden und sind zur Verwendung in nicht-viraler Gentherapie oder genetischer Impfung geeignet

### Hintergrund der Erfindung

Die Verwendung von Nukleinsäuren zur Prävention und Behandlung von Erkrankungen gewinnt durch moderne Therapieformen wie Gentherapie und genetische Impfung zunehmend an Bedeutung. Bei diesen Anwendungen werden Nukleinsäuren als nicht-virale Transfervehikel, sogenannte Vektoren, für den Transport der therapeutischen Gene in die Zielzellen genutzt. Dieser Gentransfer kann einerseits durch direkte Injektion der Nukleinsäuren in Gewebe oder Zellen oder andererseits mit Hilfe einer *Gene Gun* erfolgen. Dabei können die Nukleinsäuren als nackte DNA vorliegen oder formuliert mit Liposomen oder anderen Substanzen, die den Gentransfer oder generell die Effizienz erhöhen.

Zur Gewinnung der Nukleinsäuren macht man sich die Eigenschaft von Mikroorganismen zunutze, extra-chromosomale, zirkuläre Nukleinsäuren, wie Plasmide, Cosmide, BACs *(bacterial artificial chromosomes,* bakterielle künstliche Chromosomen) oder YACs *(yeast artificial chromosomes,* künstliche Hefe-Chromosomen) replizieren zu können.

Plasmide sind extra-chromosomale, doppelsträngige DNA-Moleküle mit einer Größe von 200 bis zu mehr als 100.000 Basenpaaren, die in der Natur sowohl in prokaryotischen als auch eukaryotischen Zellen vorkommen. Plasmide replizieren unabhängig vom Chromosom der Wirtszelle. Mit Hilfe der modernen Gentechnologie sind von den natürlich vorkommenden Spezies eine Vielzahl künstlicher Plasmide, wie pBR322 oder die pUC-Familie synthetisiert worden. In der Biotechnologie werden diese Plasmide als Klonierungsvoktoren für die rekombinante DNA-Technologie oder die Herstellung rekombinanter Proteine in Pro- und Eukaryonten verwendet. Plasmidvektoren (typische Größe < 10.000 Basenpaare) sind kleiner als die natürlichen Plasmide und bestehen aus einem Replikationssystem, einem oder mehreren Selektionsmarker-Genen und einem Klonierungsabschnitt, der den Einbau fremder DNA ermöglicht.

Cosmide sind Plasmide, die zusätzliche cos-Stellen des lambda-Bakteriophagen besitzen. Dadurch können sie bis zu 45 Kilobasenpaare (kBp) der Insert-DNA enthalten, obwohl sie selbst nur bis zu 9 kBp groß sind.

BACs und YACs sind künstliche DNA-Vektorsysteme für Klonierungsfragmente bis zu einer Größe von mehreren hundert kBp.

Extra-chromosomale DNA-Moleküle, wie Plasmide, Cosmide, BACs oder YACs liegen in verschiedenen Arten und Formen vor, die sich in Topologie und Größe unterscheiden. Wenn beispielsweise Plasmide aus Bakterienzellen isoliert werden, liegt die überwiegende Anzahl der Plasmide in einer kovalent geschlossenen, zirkulären Struktur vor, die negativ superspiralisiert ist und "ccc Form" *(covalently closed circular)* oder "Form I" genannt wird (siehe Sinden, DNA structure and function, 1994, Academic Press, San Diego).

Superspiralisierte Moleküle haben eine kompakte Struktur, wobei die DNA-Helix um sich selbst verdrillt ist. Beide Stränge sind intakt und damit kovalent geschlossen. Der Bruch *(nicking)* eines DNA-Stranges durch Nukleasen oder mechanischen Stress führt durch Verlust der Superspiralisierung zu einer offenen, zirkulären Struktur (oc Form, *open circular).* Die oc Form, die auch Form II genannt wird, ist vollständig entspannt und damit weniger kompakt. Lineare DNA-Moleküle resultieren aus dem Bruch beider Stränge an der selben Stelle oder durch Restriktion mit Endonukleasen.

Die hier beschriebenen topologischen DNA-Strukturen können zudem als äquivalente Multimer-Strukturen, wie Dimere, Trimere, Tetramere, etc. vorliegen, die in diesem Fall Koncatamere genannt werden. Koncatamere können während des Replikationsvorganges entstehen, werden aber hauptsächlich nach der Replikation durch homologe Rekombination gebildet. Die *in vivo* Bildung von Multimeren ist Wirtsstamm- und Plasmid-spezfisch. *E. coli* besitzt zahlreiche Gene, die eine Rekombination von Nukleinsäuren zu entsprechenden Multimeren ermöglichen (siehe zum Beispiel: Cohen et al., Journal of Bacteriology 167 (1986), 327-335; Kusano et al., Journal of Molecular Biology 209 (1989), 623-634; Luisi-DeLuca et al., Genetics 122 (1989), 269-278; Yannish-Perron et al., Gene 13 (1985), 103-111).

Einige Mutanten des Wildtyps weisen jedoch Veränderungen im recA-Gen auf (z.B. Mutanten mit dem Genotyp *recA*⁻ oder r*ecA1*) und sind deshalb nicht mehr in der Lage, Multimere zu bilden (siehe zum Beispiel Hanahan et al., Journal of Molecular Biology 166 (1983), 557-580). Weiterhin wird berichtet, dass das Multimer-Monomer-Verhältnis mit der Größe des integrierten Fragmentes ansteigt (siehe Summers et al., The biology of plasmids. Blackwell Science, 1996, Oxford).

Koncatamere liegen auch in linearen, entspannten oder superspiralisierten Formen vor. Bei der Behandlung mit Restriktionsenzymen werden sie in lineare Monomere fragmentiert.

Die verschiedenen Plasmidformen können durch Agarosegelelektrophorese und chromatographische Verfahren aufgetrennt werden. Bei der Agarosegelelektrophorese werden die verschiedenen Strukturen nicht direkt visualisiert, sondern in Gegenwart eines elektrischen Feldes gemäß ihrer unterschiedlichen Mobilitäten in polymeren Matrizes getrennt. Nukleinsäuren sind wegen ihres Zucker-Phosphat-Rückgrates negativ geladen und wandern deshalb im elektrischen Feld zur positiv geladenen Anode. Polymer-Matrizes, wie Agarose- oder Polyacrylamidgele bilden ein Netzwerk, welches die Beweglichkeit großer Moleküle einschränkt. Unterschiede in der Größe oder Topologie der Plasmide haben deshalb einen großen Einfluss auf das Wanderungsverhalten dieser Moleküle. Wenn eine Plasmid-DNA-Probe mit Hilfe dieser Technik analysiert wird, können die verschiedenen Banden im Agarosegel den unterschiedlichen Plasmidgrößen (Monomer, Dimer, etc.) und auch Topologien, wie ccc, oc oder lineare Plasmidmoleküle zugeordnet werden. Aus diesen Banden des Agarosegels können auch geringe Mengen der verschiedenen DNA-Strukturen isoliert werden. Eine andere Methode zur Trennung verschiedener DNA-Strukturen ist die CsCl-Gradienten-Zentrifugation. Bei dieser Methode wird das unterschiedliche Sedimentationsverhalten von unterschiedlich großen Nukleinsauremolekülen ausgenutzt Weiterhin können diese zum Teil auch durch chromatographische Methoden, wie Gelfiltration oder Größenausschluss-Chromatographie getrennt werden (siehe z.B. Green et al., Biopharm 10 (1997), 52-62).

Durch die zunehmende therapeutische Verwendung von Nukleinsäuren In Gentherapie und genetischer Impfung ist es von großem Interesse, Nukleinsäuren in großen Mengen und gleichzeitig in hoher Qualität gewinnen zu können. Um eine wirksame Gentherapie bzw. genetische Impfung zu gewährleisten, ist außerdem der erfolgreiche und effiziente Gentransfer sehr wichtig für den Erfolg des beabsichtigten Therapie-Ansatzes. Die Erhöhung der Effizienz kann dabei möglicherweise durch eine erhöhte Gendosis erzielt werden. Damit ist die Erhöhung der Gendosis pro Nukleinsäure-Molekül eine beabsichtigte Verbesserung der Vektorsysteme. Außerdem kann bei der Formulierung mit Liposomen die Größe der Nukleinsäure-Moleküle die Effizienz des Gentransfers der resultierenden Transfektionskomplexe beeinflussen.

Es besteht daher ein Bedarf nach Verfahren zur Herstellung von Nukleinsäuren in grossen Mengen und in hoher Qualität. Die auf solche Weise gewonnenen Nukleinsäuren sollten außerdem so beschaffen sein, dass sie zur Erhöhung der Effizienz des Gentransfers beitragen können.

Dies wird ermöglicht durch das erfindungsgemäße Verfahren, welches die Herstellung von homogenen Nukleinsäuremultimeren in großen Mengen und hoher Qualität für klinische Anwendungen gewährleistet.

Gegenwärtig ist kein Verfahren bekannt, welches die Herstellung von homogenen Nukleinsäure-Multimeren für pharmazeutische Zusammensetzungen für die klinische Anwendung ermöglicht. Leahy et al. (Nucleic Acids Research 25 (1997), 449-450) berichten von der Herstellung eines inhomogenen Gemisches aus verschiedenen Multimeren durch eine enzymatische Reaktion. Cohen et al. (Journal of Bacteriology 167 (1986), 327-335) beschreiben die Intrazelluläre Synthese von linearen Plasmid-Multimeren durch verschiedene *E.coli*-Mutenten. Die linearen Multimere werden dabei auch als inhomogenes Gemisch verschiedener Größen erhalten. In beiden Fällen ist keine homogene Herstellung der Multimere beabsichtigt.

BEDBROOK J R ET AL. offenbart, dass die Rekombination zwischen bakteriellen Plasmiden zur Bildung von Plasmid-Multimeren führen kann (CELL, Bd. 9, Nr. (4 PART 2, 1976, Seiten 707-716). SCHLEEF M et al. offenbaren Plasmid-DNA für phrmazeutische Anwendungen (DEVELOPMENTS IN BIOLOGICALS. SWITZERLAND 2000, Bd. 104, 2000, Seiten 25-31).

### Zusammenfassung der Erfindung

Die vorliegende, Erfindung beschreibt ein Verfahren zur Herstellung von homogenen Nukleinsäure-Multimeren für pharmazeutische Zusammensetzungen. Dabei wird die Ausgangs-DNA in einen bakteriellen Wirtsstamm transformiert, der geeignet ist, multimere Kopien der Ausgangs-DNA zu produzieren. Nach der Kultivierung dieser bakteriellen Transformante werden die DNA Multimere getrennt. Diese Trennung umfaßt die Freisetzung der DNA aus den Bakterienzellen und einen oder mehrere Schritte zur Trennung der jeweiligen Multimere von den anderen Spezies. Eine bevorzugte Trenntechnik beinhaltet die Reinigung der DNA von anderen Zellkomponenten, wie Proteine, RNA und genomische Wirtsstamm-DNA durch Anionenaustausch-Chromatographie und einer anschließenden Agarosegelelektrophorese zur Trennung der verschieden mono- und multimeren DNA-Moleküle. Aus Agarosegelen können die DNA-Moleküle extrahiert werden, z.B. durch Ausschneiden der Banden und Schmelzen der Agarose wie bei z.B. Sambrook et al. (Molecular Cloning, A Laboratory Manual, CSH Press; 2nd edition, 1989, Cold Spring Harbor, New York) beschrieben oder durch die Verwendung geeigneter Kits.

Andere Reinigungsmethoden ermöglichen die Trennung von Multimer-DNA-Molekülen durch chromatographische Verfähren, wie Gelfiltration oder Größenausschluss-Chromatographie.

Für die homogene stabile Herstellung der multimeren DNA für pharmazeutische Anwendungen wird diese in einen anderen bakteriellen Wirt transformiert, der nur diese multimere DNA produziert. Die anschließende Kultivierung und Isolierung des Multimers wird so durchgeführt, dass das gereinigte Nukleinsäure-Multimer für pharmazeutische Zusammensetzungen und Applikationen verwendet werden kann, d.h. ohne die Verwendung der toxischen Substanzen Cäsiumchlorid, Phenol, Chloroform oder Ethidiumbromid.

### Detaillierte Beschreibung der Erfindung

Gegenstand der vorliegenden Erfindung ist ein Verfahren zur Herstellung von homogenen Nukleinsaure-Multimeren für pharmazeutische Zusammensetzungen, dadurch gekennzeichnet, dass (a) die Ausgangs-DNA in einen bakteriellen Wirtsstamm transformiert wird, wobei dieser Wirtsstamm die post-replikale Rekombination der Ausgangs-Nukleinsäuremolekale zu multimeren Nukleinsäuremolekülen ermöglicht, (b) die bakterielle Transformante kultiviert wird, (c) die DNA Multimere aus der bakteriellen Transformante isoliert und getrennt werden, (d) ein abgetrenntes Multimer in einen anderen bakteriellen Wirtsstamm transformiert wird, der die post-replikale Rekombination zu multimeren Nukleinsäuremolekülen nicht ermöglicht, (e) die multimere Transformante kultiviert wird und (f) die homogenen DNA Multimere isoliert werden, wobei Schritte (e) und (f) ohne Verwendung der der toxischen Substanzen Cäsiumchlorid, Phenol, Chloroform oder Ethidiumbromid.

Die bakteriellen Wirtsstämme, die im erfindungsgemäßen Verfahren genutzt werden, können gram-positiv oder gram-negativ sein.

In einer Ausführungsform der Erfindung ist der bakterielle Wirtsstamm in Schritt (a) ein E.coli K12-Stamm mit Genotyp recA+ und der bakterielle Wirtsstamm in Schritt (d) ein E.coli K12-Stamm mit Genotyp recA- oder recA1.

E.coli-Stämme mit dem Genotyp recA+ besitzen keinerlei Mutationen in den Rekombinationsgenen und ermöglichen so eine post-replikale Rekombination von Monomeren zu dimeren Nukleinsäuremolekülen.

im Gegensatz dazu besitzen E.coli-Stämme mit den Genotypen recA- oder recA1 Mutationen im Rekombinationsgen, so dass eine Rekombination nicht mehr möglich ist. Deshalb wird in Schritt (e) des erfindungsgemäßen Verfahrens nur noch homogenes Produkt gebildet.

In einer bevorzugten Ausführungsform der Erfindung ist der bakterielle Wirtsstamm In Schritt (a) E. coli K12-JM105 (siehe zum Beispiel Yannish-Perron et al., Gene 13 (1985), 103-111) und in Schritt (d) E:coli K12-DH5α (siehe zum Beispiel Hanahan et al., Journal of Molecular Biology 166 (1983), 567-580).

Das erfindungsgemäße Verfahren ist außerdem dadurch gekennzeichnet, dass es für jede extra-chromosomale Nukleinsäure, wie Plasmide, Cosmide, YACs oder BACs, genutzt werden kann. Das efindungsgemäße Verfahren erlaubt daher die Herstellung homogener Multimere für einen großen Anwendungsbereich von Typ oder Größe der Nukleinsäuren.

In einer bevorzugten Ausführungsform der Erfindung ist die Ausgangs-DNA in Schritt (a) ein Monomer und das resultierende homogene DNA-Multimer in Schritt (f) ein ccc Dimer, oder die Ausgangs-DNA in Schritt (a) ist ein Dimer und das resultierende homogene DNA-Multimer in Schritt (f) ein ccc Trimer oder ccc Tetramer. Allgemein ist die Ausgangs-DNA in Schritt (a) ein n-mer und das resultierende homogene DNA-multimer in Schritt (f) ein (n+1)-mer oder (n+2)-mer oder (n+n)-mer.

Vor der Auftrennung der DNA-Multimere in Schritt (c) des erfindungsgemäßen Verfahrens werden die Bakterienzellen lysiert und die DNA isoliert. Die Lyse und die Isolierung erfolgen dabei nach im Stand der Technik beschrieben Verfahren, siehe beispielsweise Marquet et al. (Biopharm 8 (1995), 26-37) oder Colpan et al. (US 5990301, 1999).

Zur Auftrennung der verschiedenen Nukleinsäurestrukturen sind zahlreiche Möglichkeiten bekannt, wie CsCl-Gradienten-Zentrifugation oder chromatographische Verfahren im Anschluss an die Zelllyse. In einer bevorzugten Ausführungsform des erfindungsgemäßen Verfahrens wird die Trennung nach der Zelllyse mit Agarosegelelektrophorese, anschließendem Ausschneiden der Banden aus dem Agarosegel und der Extraktion der Nukleinsäuren aus dem Agarosestücken durchgerührt.

Überraschenderweise führt das erfindungsgemäße Verfahren zu einer Herstellung von Multimeren mit einer Homogenität von mehr als 90 %, wobei das gereinigte Multimer aus Schritt (f) fast ausschließlich in der ccc Form vorliegt. Im Gegensatz dazu führten Verfahren aus dem früheren Stand der Technik zu einer inhomogenen Multimerherstellung durch enzymatische Behandlung (Leahy et al., Nucleic Acids Research 25 (1997), 449-450) oder intra-zellulär durch *E.coli* Mutanten (Cohen et al., Journal of Bacteriology 167 (1986), 327-335).

Mit dem Verfahren der vorliegenden Erfindung können große Mengen eines homogenen Multimers gewonnen werden. Gerade für die Herstellung von Nukleinsäuren für klinische Applikationen ist eine Herstellung von großen Mengen im Gramm oder sogar Im Kilogramm- bzw. Tonnenmaßstab notwendig. Bei der hier beschriebenen Erfindung ist die Kultivierung der multimeren Transformante in Schritt (e) in jedem Herstellungsmaßstab möglich. Verfahren aus dem froheren Stand der Technik benutzen zusätzliche enzymatische Schritte für die Multimer-Herstellung, die teuer und im Maßstab limitiert sind. Darüber hinaus kann die multimere Transformante überraschenderweise über einen langen Zeitraum kultiviert werden, ohne dass ein Zerfall des Multimers in niedere inhomogene Bestandteile beobachtet wird.

in der Erfindung werden die Kultivierung in Schritt (e) und die Isolierung in Schritt (f) ohne die Verwendung toxischer Substanzen durchgerührt Dies stellt einen entscheidenden Vorteil für die Gewinnung von multimeren Nukleinsäuren in klinische Qualität gegenüber bekannten Verfahren dar.

Im erfindungsgemäßen Verfahren werden bei der Kultivierung Nährmedlen ohne tierische Inhaltsstoffe verwendet Die Isolierung und Reinigung des homogenen DNA-Multimers erfolgt durch Methoden der Zelllyse und Chromatographie, die keine toxischen Substanzen, wie beispielsweise Cäsiumchlorid, Phenol, Chloroform oder Ethidiumbromid verwenden. Außerdem werden keine anderen Kontaminanten, wie Restriktons- oder Ligase-Enzyme bei diesen Prozessschritten genutzt, um Multimere zu erzeugen. Damit ist es möglich, die homogenen multimeren Nukleinsäuren in pharmazeutischen Zusammensetzungen für Gentherapie oder Nukleinsäurevakzinierung zu verwenden. Im Gegensatz dazu verwendeten bisherige Verfahren die CsCl-Gradienten-Zentrifugation zu Reinigung des Multimers. Die Verwendung des hoch-toxischen Ethidiumbromids und Caslumchlorids schließen den Einsatz der multimeren Nukleinsäuren in der Klinik und Pharmazie jedoch aus.

Ein anderer entscheidender Vorteil der Erfindung betrifft die Tatsache, dass die Kultivierung in Schritt (e) zu großen Mengen des gewünschten Produktes führt. Andere Plasmidstrukturen, die die Produktivität der Methode vermindern würden, weil sie in zusätzlichen Reinigungsschritten vom gewünschten Multimer getrennt werden müßten, werden in vernachlässigbar geringen Mengen produziert.

In einer weiteren Ausführungsform der Erfindung wird die multimere Transformante In Schritt (d) als Glycerinkultur gelagert Dies stellt einen weiteren überraschenden Vorteil der Efindung dar. Es können somit Zellbanken der multimeren Transformante angelegt werden, die es erlauben bei der Kultivierung in Schritt (e) immer mit dem gleichen Ausgangsmaterial zu beginnen. Dieses ist bei der Herstellung von klinischen Produkten ein wichtiger Aspekt, weil nur eine reproduzierbare Herstellung die hohen Anforderungen bei der Herstellung von Pharmazeutika garantiert

In einer weiteren bevorzugten Verkörperung der Methode der Erfindung werden die Zellen nach der Kultivierung in Schritt (e) eingefroren oder gefriergetrocknet Dieses ist sehr nützlich, wenn eine sofortige Isolierung und Reinigung der homogenen Multimere nicht gewünscht ist und die Zellen längere Zeit gelagert werden sollen.

Die isolierten homogenen Nukleinsaure-Multimere können auch für pharmazeutische Zusammensetzungen verwendet werden, die für therapeutische Zwecke geeignet sind. Typische pharmazeutische Zusammensetzungen dieser Art bestehen aus Nukleinsäure (Konzentration 0.01-10 mg/mL), die in WFI (water for injection) oder anderen neutralen wäßrigen Pufferlösung gelöst ist, oder an feste Phasen wie beispielsweise Gold oder Platin gebunden ist oder inkapspliert ist oder kömplexiert (z B. mit Lipiden) ist. In einer bevorzugten Ausführungsform der Erfindung werden die. Isolierten Nukleinsäure-Multimere für nicht-virale Gentherapie oder DNA-Impfung verwendet Der Erfolg nicht-viraler Gentherapie oder DNA-Impfung ist in erster Linie von einem erfolgreichen und effizienten Gentransfer abhängig, wobei eine Erhöhung der Effizienz möglicherweise durch eine erhöhte Gendosis erzielt werden kann.

In einer weiteren bevorzugten Ausführungsförm der Erfindung ermöglicht die Verwendung des isolierten homogenen Nukleinsäure-Multimers die Erhöhung der Gendosis pro Nukleinsäuremolekül in nicht-viraler Gentherapie oder genetischer Impfung. Durch das erfindungsgemäße Verfahren kann die Größe von Nukleinsäuren für klinische Anwendungen beeinflusst werden, d.h. kleinere Nukleinsäuremoleküle können durch die Multimer-Herstellung vergrößert werden. Somit kann mit dem erfindungsgemäßen Verfahren die Dosis des therapeutischen Gens pro eingebrachtem Nukleinsäuremolekül bei therapeutischer Anwendung erhöht werden.

Die Erfindung wird weiterhin durch die folgenden Abbildungen erläutert:

### Abbildungen

Die Abbildungen zeigen
- **Abbildung 1:**: 0,8 % Agarosegelelektrophorese des Plasmids pUT649 aus *E. coli* DH5α.
- **Abbildung 2:**: 0,8 % Agarosegelelektrophorese des Plasmids pUT649 (Spuren 1-4), aus *E. coli* K12JM105 isoliert (Spur M: λ-DNA, BStE **II** verdaut).
- **Abbildung 3:**: 0,8 % Agarosegelelektrophorese des Plasmids pUT649 (Spuren 1-4) und eine mit UV-Licht bestrahlte Probe (Spur 5), isoliert aus *E*. *coli* K12JM105 zur Gelextraktion (Spur M: λ-DNA, BStE **II** verdaut).
- **Abbildung 4:**: 0,8 % Agarosegelelektrophorese des dimeren pUT649 Plasmids, isoliert aus *E. coli* DH5α ((Spuren 1 und 2).
- **Abbildung 6:**: 0,8 % Agarosegelelektrophorese des tetrameren pUT649 Plasmids, isoliert aus *E. coli* DH5α.

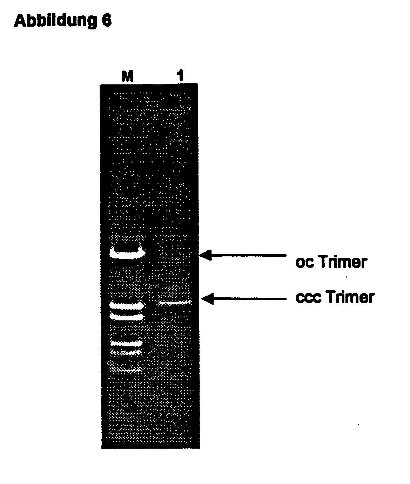

- **Abbildung 6:**: 0,8 %Agarosegelelektrophorese des trimeren pUT649 Plasmids, isoliert aus *E. coli* DH5α.

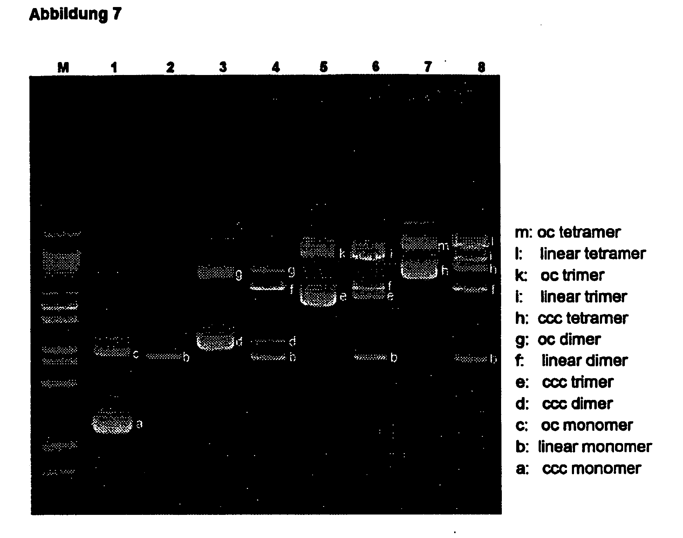

- **Abbildung 7:**: 0,8 % Agarosegeleleictrophorese verschiedener pUT649 Multimere,
Isoliert aus *E.coli* DH5α:
Spur M: λ-DNA, BstE II verdaut.
Spur 1: pUT649 Monomer.
Spur 2: pUT649 Monomer, vollständig Sall verdaut, linear.
Spur 3: pUT649 Dimer.
Spur 4: pUT649 Dimer, partiell Sal I verdaut.
Spur 5: pUT649 Trimer.
Spur 6: pUT649 Trimer, partiell Sall verdaut.
Spur 7: pUT649 Tetramer.
Spur 8: pUT649 Tetramer, partiell Sall verdaut.

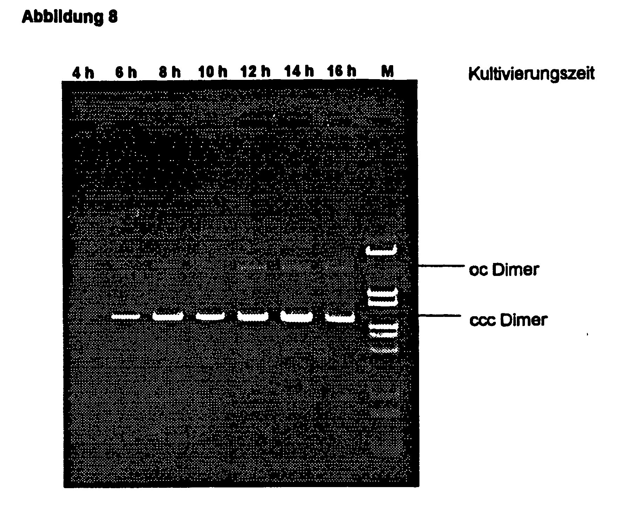

- **Abbildung 8:**: 0,8 % Agarosegelelektrophorese von aufgereinigten pUT649-Dimer DNA Proben, isoliert aus *E*. *coli* DH5α zu verschiedenen Kultivierungszeitpunkten. Die Kultivierung wurde im 7 L Bioreaktor durchgeführt.

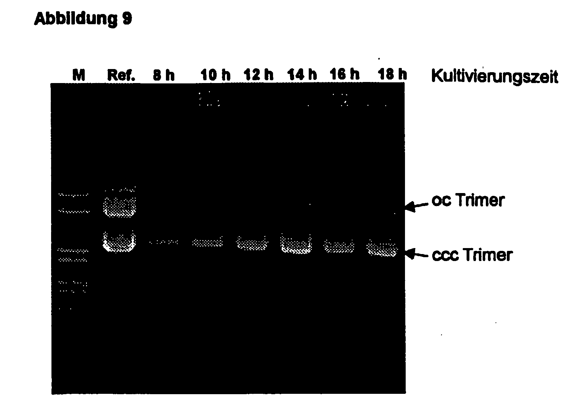

- **Abbildung 9:**: 0,8 % Agarosegelelektrophorese von aufgereinigten pUT649-Trimer DNA Proben, isoliert aus *E. coli* DH5α zu verschiedenen Kultivierungszeitpunkten. Die Kultivierung wurde im 7 L Bioreaktor durchgeführt.

Die folgenden Bespiele dienen der Veranschaulichung der Erfindung und sind nicht in beschränkender Weise aufzufassen.

### Beispiele

### Beispiel 1: Homogene Herstellung eines 9,2 kbp großen ccc-Dimer Plasmids ausgehend vom 4,6 kbp großen Monomer.

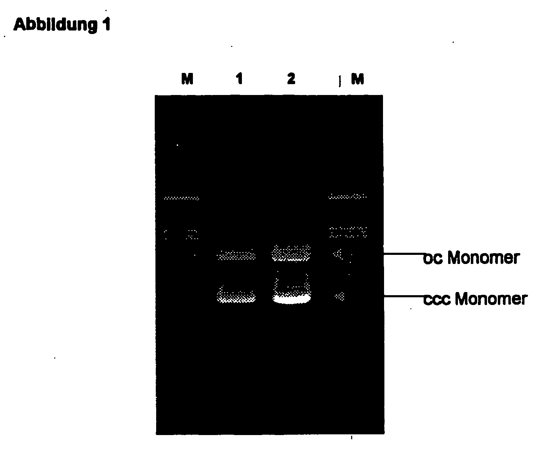

Das Verfahren zur Herstellung des homogenen ccc-Dimers des 4,6 kbp großen Plasmids pUT649 (Eurogentec, Liege, Belgien) wurde mit den Stämmen *E. coli* K12JM105 (DSMZ Nr. 3949) und *E. coli* DH5α (Clontech, Heidelberg, Deutschland, Cat.-Nr. C2007-1) durchgeführt. Die Ausgangs-Plasmid-DNA (Abbildung 1) wurde in kompetente Zellen von *E. coli* K12JM105 transformiert.

Ein Volumen von 5 µL Plasmid DNA in TE-Puffer (konzentration ca. 100 µg mL⁻¹) wurde zu 100 µL kompetenter *E. coli* K12JM105 Zellen gegeben. Die Probe wurde 20 Minuten auf Eis inkubiert, für 3 Minuten bei 42°C inkubiert und weitere 5 Minuten auf Eis gekühlt. Ein Volumen von 1 mL vorgewärmtes Luria-Bertani Medium (LB Medium) wurde zu der Transformationsprobe gegeben und nach 45 minütiger Inkubation bei 37°C die Zellen in einer Tischzentrifuge bei 7000 min⁻¹ abgetrennt. Vom Überstand wurden 900 µL abgenommen und verworfen. Die Zellen wurden im Restvolumen resuspendiert. Ein Volumen von 50 µL wurde auf Ampicillin-LB-Agarplatten (100 µg mL⁻¹ Ampicillin) ausplattiert. Nach 24 stündiger Inkubation bei 37°C wurden Einzelkolonien dieser Platte auf einer neuen Ampidillin-LB-Agarptatte ausplattiert Nach weiteren 24 Stunden Inkubationszeit wurden zwei Schüttelkolben (300 mL) gefüllt mit 30 mL LB Medium und Ampicillin mit Einzelkoionien dieser Platten Inokuliert und für 24 Stunden bei 37°C auf einem Laborschüttler bei 120 min⁻¹ bebrütet. Aus 3 mL Kulturvolumen wurde die Plasmid DNA mittels z.B. QIAGEN Miniprep Kits (Tip-20) gemäß den Vorschriften des Kit-Herstellers isoliert. Die unbehandelte Plasmid DNA wurde mit einem 0,8 %igen Agarosegel analysiert (300 ng DNA pro Tasche, Agarose von Bio-Rad, München, Deutschland). Die elektrophoretische Trennung erfolgte bei einer elektrischen Feldstärke von E = 50 V cm⁻¹. Nach 1 Stunde wurde das Gel in Ethidiumbromid Lösung (0,5 mg L⁻¹ in TAE-Puffer) gefärbt und 10 Minuten in Wasser gewaschen. Die DNA Banden wurden unter einem UV-Illuminator (λ = 312 nm) sichtbar gemacht. Die Dokumentation erfolgte mit dem Geldokumentationssystem E.A.S.Y. (Herolab, Deutschland).

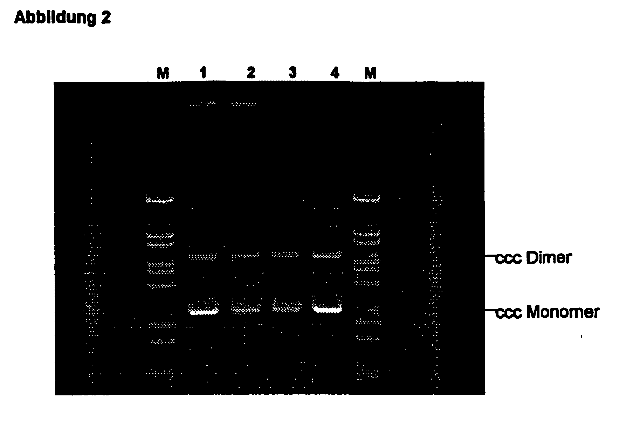

Abbildung 2 zeigt die Verteilung der Plasmidformen der isolierten Proben. Neben der ccc-Monomer Form wurde auch die ccc-Dimer Form von *E. coli* K12JM105 repliziert.

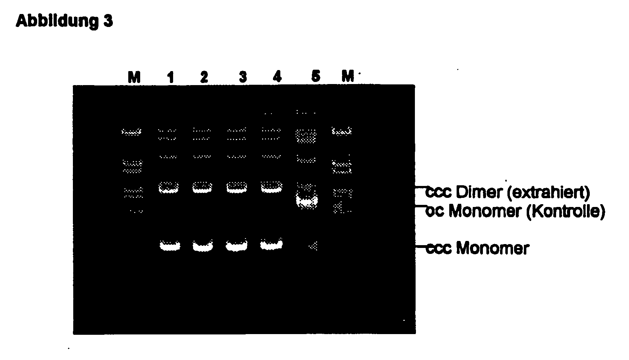

Ein präparatives Agarosegel (low-melting Agarose, Bio-Rad) wurde wie oben beschrieben mit einer größeren Menge (1 µg) angefertigt. Zur Unterscheidung von ccc-Dimer und oc-Monomer wurde eine oc-Monomer Probe (erhalten durch Bestrahlung einer ccc-Monomerprobe) parallel analysiert (Abbildung 3). Die ccc-Dimerbande wurde aus diesem Gel mit einem Gelextraktionskit, z.B. QIAEX II (QIAGEN, Hilden, Deutschland), gemäß den Herstellerangaben extrahiert.

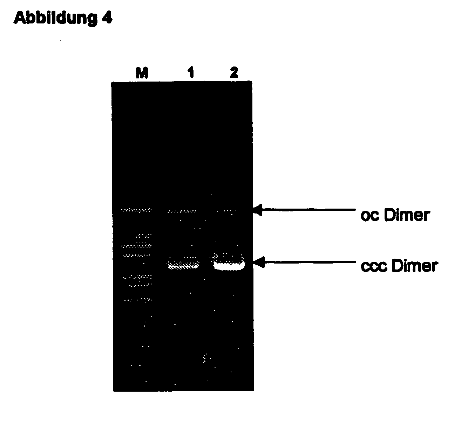

1 µg extrahierte Plasmid DNA wurde, wie oben beschrieben, in kompetente E. coli DH5α Zellen transformiert und auf ampicillinhaltigen Agarplatten kultiviert. Ein Volumen von 5 mL LB Medium mit Ampicillin (100 µg mL⁻¹) wurde mit einer Einzelkolonie dieser Platte inokuliert und über Nacht bei 37°C inkubiert. Aus 3 mL Kulturvolumen wurde die Plasmid DNA isoliert (QIAGEN Miniprep Kit). Die Isolierte DNA wurde auf einem 0,8 %igem Agarosegel analysiert, welches das homogene ccc-Dimer Plasmid zeigt (Abbildung 4). Glycerinkulturen zur Langzeit-Lagerung bei -80 °C wurden aus einer Übernachtkultur des pUT 649-Dimer produzierenden Klons in ampicillinhaltigem LB-Medium angelegt. Hierzu wurden 870 µL Kulturvolumen zu 130 µL 88 %igem Glycerin gegeben, gemischt, in flüssigem Stickstoff schockgefroren und bei -80 °C gelagert.

### Beispiel 2: Homogene Herstellung eines 18,4 kbp großen ccc-Tetramer Plasmids ausgehend vom 9,2 kbp großen Dimer.

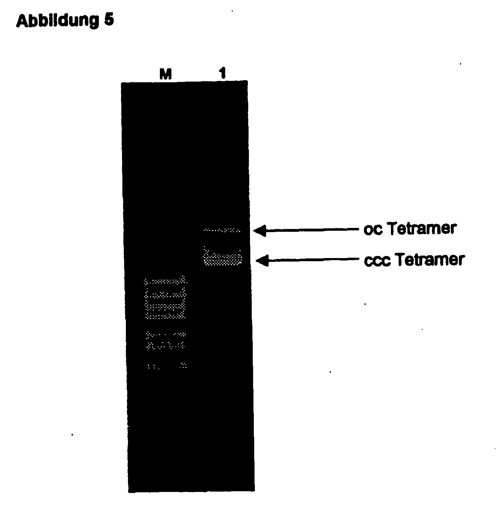

Zur Herstellung des 18,4 kbp großen ccc-Tetramer des Plasmids pUT 649 wurde das ccc-Dimer dieses Plasmids, dessen Herstellung in Beispiel 1 beschrieben ist, in *E. coli* K12JM105 transformiert. Der so hergestellte Klon wurde auf ampicillinhaltigen LB-Agarplatten selektioniert. Ein Volumen von 30 mL LB Medium wurde mit einer Einzelkolonie dieser Platten inokuliert und über Nacht bei 37 °C inkubiert. Die Plasmid DNA wurde aus 3 mL Kulturvolumen isoliert und auf einem 0,8 %igen Agarosegel analysiert Die ccc-Tetramerform wurde aus dem Gel mittels QIAEX II Kit extrahiert und in *E. coli* DH5α transformiert. Der transformierte Klon wurde auf einer ampicillinhaltigen LB-Agarplatte über Nacht kultiviert Eine Schüftelkolbenkultur mit ampicillinhalügem LB-Medium wurde mit einer Einzelkolonie dieser Platte inokuliert Nach Kultivierung über Nacht wurde die ccc-Tetramer Plasmid DNA aus je 3 mL Kulturvolumen mit QIAGEN Miniprep isoliert und auf einem Agarosegel analysiert (Abbildung 5). Das Agarosegel zeigt, dass ccc-Tetramer Plasmid DNA hoher Homogenität produziert wurde.

### Beispiel 3: Homogene Herstellung eines 13,8 kbp großen ccc-Trimer Plasmids ausgehend vom 9,2 kbp großen Dimer.

Zur Herstellung des 13,8 kbp großen ccc-Trimers des Plasmids pUT 649 wurde das ccc-Dimer dieses Plasmids, dessen Herstellung in Beispiel 1 beschrieben ist, in *E. coli* K12JM105 transformiert. Der so hergestellte Klon wurde auf ampicillinhaltigen LB-Agarplatten siblektioniert. Ein Volumen von 30 mL LB Medium wurde mit einer Einzelkolonie dieser Platten inokuliert und über Nacht bei 37 °C inkubiert. Die Plasmid DNA wurde aus 3 mL Kulturvolumen isoliert und auf einem 0,8 %igen Agarosegel analysiert. Die ccc-Trimerform wurde aus dem Gel mittels QIAEX II Kit extrahiert und in *E. coli* DH5α transformiert. Der transformierte Klon wurde auf einer ampicillinhaltigen LB-Agarplatte über Nacht kultiviert. Eine Schüttelkolbenkultur mit ampicillinhaltigem LB-Medium wurde mit einer Einzelkolonie dieser Platte inokuliert. Nach Kultivierung über Nacht wurde die ccc-Trimer Plasmid DNA aus je 3 mL Kulturvolumen mit QIAGEN Miniprep isoliert und auf einem Agarosegel analysiert. Das Agarosegel in Abbildung 6 zeigt die Herstellung eines homogenen ccc-Trimer Plasmids. Zur Langzeit-Lagerung bei -80 °C wurden Glycerinkulturen angefertigt

### Beispiel 4: Identifizierung der Plasmid Multimere durch partielle Restriktion

Die Herstellung verschiedener homogener Plasmid Multimere ist in den Beispielen 1 bis 3 beschrieben. Diese Multimere wurden durch das spezifische Restriktionsenzym Sal 1 (MBI Fermentas, St. Leon-Rot, Deutschland) partiell linearisiert (enzymatische Aktivitäten: 2 U/µL: 1 U/µL: 0,5 U/µL: 0,25 U/µL). Ein Volumen von je 5 µL Enzymlösung, 5 µL Enzympuffer, 5 µL Plasmid DNA und 35 µL Wasser wurden gemischt und bei 37 °C für 8 Minuten inkubiert. Das Enzym wurde durch 5 minütiges Erhitzen des Restriktionsansatzes auf 65°C desaktiviert Je eine dieser partiell verdauten Proben, die lineare Multimerformen aufwiesen, wurde in einem Agarosegel analysiert

Abbildung 7 beschreibt die Bildung linearer Formen des Monomers, Dimers, Trimers und Tetramers durch partielle Restriktion.

### Beispiel 5: Herstellung von homogenem ccc Plasmid Dimer im 7 L Bioreaktor im Großmaßstab

Zur Herstellung des Plasmid Dimers im Großmaßstab wurde eine Inokulationskultur von 200 mL sterilem, halbdefiniertem Medium mit Sojapepton, Hefeextrakt, Glycerin und 100 µg mL⁻¹ Ampicillin in einem 1 L Schüftelkolben mit 200 µL Glycerinkultur des Klons pUT 649-Dimer in E. *coli* DH5a aus Beispiel 1 beimpft. Nach 8 stündiger Inkubation bei 37°C auf einem Laborschüttler wurde ein 7 L Bioreaktor (MBR, Wetzikon Schweiz), gefüllt mit 5 L sterilem halbdefinierten Medium, mit 50 mL der Inokulabonskultur beimpft. Die Kultivierung im Bioreaktor wurde bei 37 °C und 0,2 bar durchgeführt Luftzufuhr erfolgte mit 5 L min⁻¹ und der pH wurde auf 7,0 durch Phosphorsäure und/oder NaOH geregelt. Die Rührerfrequenz betrug zu Beginn der Kultivierung 150 min⁻¹ und wurde um 2 % erhöht, sobald die Gelöstsauerstoflkonzentration einen Sollwert von 40 % unterschritten hatte. Im Abstand von 2 h wurden Inprozess-Kontrollen entnommen. Abbildung 8 zeigt ein Agarosegel dieser isolierten pUT 649-Dimerproben. Während des gesamten Kultivienrngsprozesses wurde das ccc-Dimer Plasmid von den Bakterienzellen in hoher Homogenität produziert Die Herstellung des ccc-Dimers erfolgte in den Bakterienzellen ohne Rückbildung monomerer Strukturen. Am Ende der Kultivierung konnten 130 mg homogene ccc-Dimer Plasmid DNA isoliert werden.

### Beispiel 6: Herstellung von homogenem ccc Plasmid Trimer im 7 L Bioreaktor im Großmaßstab

Eine weitere Kultivierung im 7 L Bioreaktor wurde zur Herstellung des pUT649 Trimers im Großmaßstab durchgeführt. Hierzu wurde eine Glycennkultur vom pUT649-Trimer in E. *coli* DH5α. dessen Herstellung in Beispiel 3 beschrieben ist, verwendet. Die Kultivierungsbedingungen für die Inokulabonskultur und den Bioreaktor wurden bereits in Beispiel 5 beschrieben. Das Agarosegel der Plasmidproben aus der Prozesskontrolle in Abbildung 9 zeigt, dass das cco-Trimer mit hoher Homogenität produziert wurde. Die Bildung anderer Plasmidstrukturen konnte nicht beobachtet werden. Am Ende der Kultivierung nach 18 Stunden konnten 110 mag ccc-Trimer Plasmid DNA isoliert werden.

## Patentansprüche

1. Ein Verfahren zur Herstellung von homogenen Nukleinsäure-Multimeren für pharmazeutische Zusammensetzungen, umfassend die folgenden Schritte:
(a) Transformieren von Ausgangs-DNA in einen bakteriellen Wirtsstamm, wobei dieser Wirtsstamm die post-replikale Rekombination der Ausgangs-Nukleinsäuremoleküle zu multimeren Nukleinsäuremolekülen ermöglicht,
(b) Kultivieren der bakteriellen Transformante,
(c) Isolieren der DNA-Multimere aus der bakteriellen Transformante und Auftrennen der erhaltenen DNA-Multimere,
(d) Transformieren der aufgetrennten DNA-Multimere in einen anderen bakteriellen Wirtsstamm, wobei dieser Wirtsstamm die post-replikale Rekombination zu multimeren Nukleinsäuremolekülen nicht ermöglicht,
(e) Kultivieren der multimeren Transformante ohne die Verwendung der toxischen Substanzen Cäsiumchlorid, Phenol, Chloroform oder Ethidiumbromid,
(f) Isolieren des homogenen DNA-Multimers ohne die Verwendung der toxischen Substanzen Cäsiumchlorid, Phenol, Chloroform oder Ethidiumbromid.

2. Ein Verfahren gemäss Anspruch 1, wobei der bakterielle Wirtsstamm, in Schritt (a) ein E.coli K12-Stamm mit Genotyp *recA⁺* ist.

3. Ein Verfahren gemäss Anspruch 2, wobei der bakterielle Wirtsstamm in Schritt (a) E.coli K12-JM105 ist.

4. Ein Verfahren gemäss Anspruch 1, wobei der bakterielle Wirtsstamm in Schritt (d) ein E.coli K12-Stamm mit Genotyp *recA*⁻ oder *recA1* ist.

5. Ein Verfahren gemäss Anspruch 4, wobei der bakterielle Wirtsstamm in Schritt (d) *E.coli* K12-DH5α ist.

6. Ein Verfahren gemäss einem der Ansprüche 1 bis 5, wobei die Nukleinsäuren ausgewählt sind aus der Gruppe bestehend aus Plasmiden, Cosmiden, BACs oder YACs.

7. Ein Verfahren gemäss Anspruch 1, wobei die Ausgangs-DNA in Schritt (a) ein Monomer und das homogene DNA-Multimer in Schritt (f) ein Dimer ist.

8. Ein Verfahren gemäss Anspruch 1, wobei die Ausgangs-DNA in Schritt (a) ein Dimer und das homogene DNA-Multimer in Schritt (f) ein Trimer oder Tetramer ist.

9. Ein Verfahren gemäss Anspruch 1, wobei die Ausgangs-DNA in Schritt (a) ein n-mer ist und das homogene DNA-Multimer in Schritt (f) ein (n+1)-mer oder (n+2)-mer oder (n+n)-mer ist.

10. Ein Verfahren gemäss Anspruch 1, wobei die Trennung der DNA-Multimere in Schritt (c) durch Elektrophorese nach der Zelllyse durchgeführt wird.

11. Ein Verfahren gemäss Anspruch 1, wobei die Trennung der DNA-Multimere in Schritt (c) durch eines oder mehrere chromatographische Verfahren nach der Zelllyse erfolgt.

12. Ein Verfahren gemäss einem der Ansprüche 1 bis 11, wobei die Homogenität des isolierten DNA-Multimers in Schritt (f) mehr als 90 % beträgt.

13. Ein Verfahren gemäss einem der Ansprüche 1 bis 12, wobei das isolierte DNA-Multimer zu mehr als 90 % in der ccc Form vorliegt.

14. Ein Verfahren gemäss einem der Ansprüche 1 bis 13 zur Herstellung von homogenen Nukleinsäure-Multimeren für pharmazeutische Zusammensetzungen.

15. Ein Verfahren gemäss einem der Ansprüche 1 bis 14, wobei die multimere Transformante in Schritt (d) als Glycerinkultur bei -80°C gelagert wird.

16. Ein Verfahren gemäss einem der Ansprüche 1 bis 15, wobei die Zellen nach der Kultivierung in Schritt (e) eingefroren oder gefriergetrocknet werden, bevor das homogene DNA-Multimer isoliert wird.

17. Ein Verfahren gemäss Anspruch 14, wobei das isolierte homogene Nukleinsäure-Multimer für nicht-virale Gentherapie oder genetische Impfung verwendet wird.

18. Ein Verfahren gemäss Anspruch 17, wobei die Verwendung des isolierten homogenen Nukleinsäure-Multimers für nicht-virale Gentherapie oder genetische Impfung die Gendosis pro Nukleinsäure-Molekül erhöht.

## Claims

1. A method for producing homogeneous nucleic acid multimers for pharmaceutical compositions, the method comprising the steps:
(a) transforming a DNA of origin into a bacterial host strain, wherein said host strain enables the post-replicative recombination of the nucleic acid molecules of origin into multimeric nucleic acid molecules;
(b) cultivating the bacterial transformant;
(c) isolating the DNA multimers from the bacterial transformant and separating the obtained DNA multimers;
(d) transforming of the separated DNA multimers into another bacterial host strain, wherein said other host strain does not enable the post-replicative recombination into multimeric nucleic acid molecules;
(e) cultivating the multimeric transformant without the use of the toxic substances cesium chloride, phenol, chloroform, or ethidium bromide;
(f) isolating the homogeneous DNA multimers without the use of the toxic substances cesium chloride, phenol, chloroform, or ethidium bromide.

2. The method of claim 1, wherein the bacterial host strain in step (a) is an *E*. *coli* K12 strain having the genotype *recA⁺.*

3. The method of claim 2, wherein the bacterial host strain in step (a) is E. *coli* K12-JM105.

4. The method of claim 1, wherein the bacterial host strain in step (d) is an *E*. *coli* K12 strain having the genotype *recA⁻* or *recA1.*

5. The method of claim 4, wherein the bacterial host strain in step (d) is E. *coli* K12-DH5α.

6. The method of any of the claims 1 to 5, wherein the nucleic acids are selected from the group consisting of plasmids, cosmids, BACs, or YACs.

7. The method of claim 1, wherein the DNA of origin in step (a) is a monomer and the homogeneous DNA multimer in step (f) is a dimer.

8. The method of claim 1, wherein the DNA of origin in step (a) is a dimer and the homogeneous DNA multimer in step (f) is a trimer or tetramer.

9. The method of claim 1, wherein the DNA of origin in step (a) is an n-mer and the homogeneous DNA multimer in step (f) is an (n+1)-mer, an (n+2)-mer, or an (n+n)-mer.

10. The method of claim 1, wherein the separation of the DNA multimers in step (c) is performed by electrophoresis after cell lysis.

11. The method of claim 1, wherein the separation of the DNA multimers in step (c) is performed by one or more chromatographic methods after cell lysis.

12. The method of any of the claims 1 to 11, wherein the homogeneity of the isolated DNA multimers in step (f) is more than 90%.

13. The method of any of the claims 1 to 12, wherein the isolated DNA multimer is present to more than 90% in the ccc form.

14. The method of any of the claims 1 to 13 for producing homogeneous nucleic acid multimers for pharmaceutical compositions.

15. The method of any of the claims 1 to 14, wherein the multimeric transformant in step (d) is stored as a glycerin stock at -80°C.

16. The method of any of the claims 1 to 15, wherein the cells are frozen or lyophilized after cultivation in step (e) and before isolating the homogeneous DNA multimer.

17. The method of claim 14, wherein the isolated homogeneous nucleic acid multimer is used for non-viral gene therapy or genetic vaccination.

18. The method of claim 17, wherein the use of the isolated homogeneous nucleic acid multimer for non-viral gene therapy or genetic vaccination increases the gene dose per nucleic acid molecule.

## Revendications

1. Procédé de production de multimères d'acides nucléiques homogènes destinés à des compositions pharmaceutiques, le procédé comprenant les étapes suivantes .
(a) la transformation d' un ADN d'origine dans une souche bactérienne hôte, dans laquelle ladite souche hôte permet la recombinaison post-réplication des molécules d'acides nucléiques d'origine en molécules multimères d'acides nucléiques ;
(b) la culture des bactéries transformées ;
(c) l'isolement des multimères d'ADN provenant des bactéries transformées et la séparation des multimères d'ADN obtenus ;
(d) la transformation des multimères d'ADN séparés dans une autre souche bactérienne hôte, dans laquelle ladite autre souche hôte ne permet pas la recombinaison post-réplication en molécules multimères d'acides nucléiques ;
(e) la culture du transformant multimère sans utiliser les substances toxiques chlorure de césium, phénol, chloroforme ou bromure d'éthidium ;
(f) l'isolement des multimères d'ADN homogènes sans utiliser les substances toxiques chlorure de césium, phénol, chloroforme ou bromure d'éthidium.

2. Procédé selon la revendication 1, dans lequel la souche bactérienne hôte dans l'étape (a) est une souche E. *coli* K12 dont le génotype est *recA⁺*.

3. Procédé selon la revendication 2, dans lequel la souche bactérienne hôte dans l'étape (a) est *E*. *coli* K12-JM105.

4. Procédé selon la revendication 1, dans lequel la souche bactérienne hôte dans l'étape (d) est une souche *E. coli* K12 dont le génotype est *recA⁻* ou *recA1.*

5. Procédé selon la revendication 4, dans lequel la souche bactérienne hôte dans l'étape (d) est *E. coli* K12-DH5α.

6. Procédé selon l'une quelconque des revendications 1 à 5, dans lequel les acides nucléiques sont choisis dans le groupe constitué par les plasmides, les cosmides, les BAC ou les YAC.

7. Procédé selon la revendication 1, dans lequel l'ADN d'origine dans l'étape (a) est un monomère et le multimère d'ADN homogène dans l'étape (f) est un dimère.

8. Procédé selon la revendication 1, dans lequel l'ADN d'origine dans l'étape (a) est un dimère et le multimère d'ADN homogène dans l'étape (f) est un trimère ou un tétramère.

9. Procédé selon la revendication 1, dans lequel l'ADN d'origine dans l'étape (a) est un n-mère et le multimère d'ADN homogène dans l'étape (f) est un (n+1)-mère, un (n+2)-mère ou un (n+n)-mère.

10. Procédé selon la revendication 1, dans lequel la séparation des multimères d'ADN dans l'étape (c) est réalisée par électrophorèse après lyse cellulaire.

11. Procédé selon la revendication 1, dans lequel la séparation des multimères d'ADN dans l'étape (c) est réalisée par une ou plusieurs méthodes chromatographiques après lyse cellulaire.

12. Procédé selon l'une quelconque des revendications 1 à 11, dans lequel l'homogénéité des multimères d'ADN isolés dans l'étape (f) est supérieure à 90 %.

13. Procédé selon l'une quelconque des revendications 1 à 12, dans lequel le multimère d'ADN isolé est présent à plus de 90 % sous la forme ccc.

14. Procédé selon l'une quelconque des revendications 1 à 13, pour produire des multimères d'acides nucléiques homogènes destinés à des compositions pharmaceutiques.

15. Procédé selon l'une quelconque des revendications 1 à 14, dans lequel le transformant multimère dans l'étape (d) est stocké dans une base de glycérine à -80°C.

16. Procédé selon l'une quelconque des revendications 1 à 15, dans lequel les cellules sont congelées ou lyophilisées après culture dans l'étape (e) et avant l'isolement du multimère d'ADN homogène.

17. Procédé selon la revendication 14, dans lequel le multimère d'acide nucléique homogène isolé est utilisé en thérapie génique non virale ou en vaccination génétique.

18. Procédé selon la revendication 17, dans lequel l'utilisation du multimère d'acide nucléique homogène isolé en thérapie génique non virale ou en vaccination génétique augmente la dose de gène par molécule d'acide nucléique.
